# EUROPEAN PATENT APPLICATION

(11) **EP 2 138 115 A1**
(43) Date of publication of application: **30.12.2009**
(21) Application number: 09251615.2
(22) Date of filing: 22.06.2009
(51) Int. Cl.: A61B 17/34

(54) **Surgical access instrument including a valve with dynamic fluid**

(30) Priority: 26.06.2008 US 75849 P; 18.05.2009 US 467417
(71) Applicant: Tyco Healthcare Group LP, North Haven, CT 06473 (US)
(72) Inventor: Rockrohr, Brian, Waterbury, CT 06705 (US)
(74) Representative: Soames, Candida Jane

(57) **Abstract**

A surgical access instrument (10) for use during the course of a minimally invasive surgical procedure, includes an access member (20) defining a longitudinal axis and having a longitudinal opening for reception and passage of a surgical object, a valve membrane (100) mounted to the access member defining at least one internal cavity (106) and permitting passage of the surgical object and a dynamic fluid ("F") disposed within the internal cavity. The dynamic fluid is adapted to transition from a first state to a second state upon exposure thereof to a stimulus provided by a stimulation member (40) to assist in stabilizing the surgical object relative to the access member. In its first state, the fluid defines a first set of physical attributes, including but not being limited to viscosity and density, that allow for the displacement of the fluid, whereas in the second state, the fluid defines a second, dissimilar set of physical attributes, e.g., a greater viscosity and a greater density, that substantially limit displacement of the fluid. The fluid may be an electrorheological fluid, a magnetorheological fluid or a ferrofluid. The stimulus may be an electrical field or a magnetic field.

## Description

### CROSS REFERENCE TO RELATED APPLICATION

The present application claims the benefit of and priority to U.S. Provisional Application Serial No. 61/075,849, filed on June 26, 2008, the entire contents of which are incorporated herein by reference.

### BACKGROUND

### 1. Technical Field

The present disclosure relates to a surgical access instrument for use during the course of a minimally invasive surgical procedure. In particular, this disclosure relates to a valve adapted for the sealed reception of a surgical object and for use with a surgical access instrument.

### 2. Background of the Related Art

Today, many surgical procedures are performed through access devices such as trocar and cannula assemblies. These devices incorporate narrow tubes or cannula percutaneously inserted into a patient's body, through which one or more surgical objects may be introduced and manipulated during the course of the procedure. Generally, such procedures are referred to as "endoscopic", unless performed on the patient's abdomen, in which case the procedure is referred to as "laparoscopic". Throughout the present disclosure, the term "minimally invasive" should be understood to encompass both endoscopic and laparoscopic procedures.

Generally, during minimally invasive procedures, prior to the introduction of a surgical object into the patient's body, insufflation gasses are used to enlarge the area surrounding the target surgical site to create a larger, more accessible work space. Accordingly, the maintenance of a substantially fluid-tight seal along the central opening of the access device in the presence of the surgical object is desirable so as to prevent the escape of the insufflation gases and the deflation or collapse of the enlarged surgical work space. To this end, surgical access devices generally incorporate a valve.

During the course of a minimally invasive procedure, once the surgical object, or objects, have been positioned within the access device in a particular orientation, it may be desirable to maintain that orientation for a period of time, while preserving the sealed formed therewith. While there are many varieties of surgical valves known in the art, a continuing need exists for a surgical valve that is adapted to form a substantially fluid-tight seal with a surgical object and limit any undesirable movement thereof.

### SUMMARY

Accordingly, the present disclosure relates to a surgical access instrument for use during the course of a minimally invasive surgical procedure. The access instrument includes an access member defining a longitudinal axis and having a longitudinal opening for reception and passage of a surgical object; a valve membrane mounted to the access member defining at least one internal cavity and permitting passage of the surgical object and a dynamic fluid disposed within the internal cavity. The dynamic fluid is adapted to transition from a first state to a second state upon exposure thereof to a stimulus to assist in stabilizing the surgical object relative to the access member. A stimulation member may be in communication with the dynamic fluid. The stimulation member is adapted to selectively generate the stimulus. The stimulation member may be adapted to selectively generate an electrical field and/or a magnetic field.

The dynamic fluid may be selected from the group consisting of an electrorheological fluid, a magnetorheological fluid, and a ferrofluid. The dynamic fluid may define a first viscosity in the first state and a second viscosity in the second state with the second viscosity being greater than the first viscosity.

The valve membrane may include an aperture extending therethrough. The valve membrane may comprise a semi-resilient material such that the surgical valve may resiliently transition between first and second conditions upon the insertion of at least one surgical object into the aperture. The aperture of the valve membrane may define a first transverse dimension in the first condition and a second transverse dimension in the second condition. The second transverse dimension is greater than the first transverse dimension. The valve membrane may define a substantially torroidal configuration. The valve membrane may be adapted to apply a force to the surgical object subsequent to the insertion thereof into the aperture when the dynamic fluid is in the second state such that lateral movement of the surgical object with respect to the longitudinal axis is substantially limited.

A method of performing a minimally invasive surgical procedure is also disclosed. The method includes the steps of:
inserting a surgical access member into a percutaneous access point within tissue to gain access to an underlying body cavity;
introducing a surgical object within the surgical access member and through a valve disposed within the access member, the valve including at least one internal cavity configured to retain dynamic fluid;
stimulating the dynamic fluid to cause the dynamic fluid to transition from a first state having a first viscosity to a second state having a second viscosity greater than the first viscosity to assist in stabilizing the surgical object relative to the longitudinal axis; and
performing a procedure with the surgical object.

The invention also describes a surgical access member for use in a method of performing minimally invasive surgical procedure wherein the method comprises the steps of:
inserting the surgical access member into a percutaneous access point within tissue to gain access to an underlying body cavity;
introducing a surgical object within the surgical access member and through a valve disposed within the access member, the valve including at least one internal cavity configured to retain dynamic fluid;
stimulating the dynamic fluid to cause the dynamic fluid to transition from a first state having a first viscosity to a second state having a second viscosity greater than the first viscosity to assist in stabilizing the surgical object relative to the longitudinal axis; and
performing a procedure with the surgical object.

The invention may be described by reference to the following numbered paragraphs:-

A surgical access instrument for use during the course of a minimally invasive surgical procedure, which comprises:
an access member defining a longitudinal axis and having a longitudinal opening for reception and passage of a surgical object;
a valve membrane mounted to the access member, the valve membrane comprising a biocompatible material and defining at least one internal cavity, the valve membrane permitting passage of the surgical object; and
a dynamic fluid disposed within the internal cavity, the dynamic fluid being adapted to transition from a first state to a second state upon exposure thereof to a stimulus to assist in stabilizing the surgical object relative to the access member.

The surgical access instrument of paragraph [0012], including a stimulation member in communication with the dynamic fluid, the stimulation member being adapted to selectively generate the stimulus.

The surgical access instrument of paragraph [0013], wherein the stimulation member is adapted to selectively generate an electrical field.

The surgical access instrument of paragraph [0013], wherein the stimulation member is adapted to selectively generate a magnetic field.

The surgical access instrument of paragraph [0012], wherein the dynamic fluid is selected from the group consisting of an electrorheological fluid, a magnetorheological fluid, and a ferrofluid.

The surgical access instrument of paragraph [0012], wherein the dynamic fluid defines a first viscosity in the first state and a second viscosity in the second state, the second viscosity being greater than the first viscosity.

The surgical access instrument of paragraph [0012], wherein the valve membrane includes an aperture extending therethrough, the valve membrane comprising a semi-resilient material such that the surgical valve may resiliently transition between first and second conditions upon the insertion of at least one surgical object into the aperture, the aperture defining a first transverse dimension in the first condition and a second transverse dimension in the second condition, the second transverse dimension being greater than the first transverse dimension.

The surgical access instrument of paragraph [0018], wherein the valve membrane defines a substantially torroidal configuration.

The surgical access instrument of paragraph [0018], wherein the valve membrane is adapted to apply a force to the surgical object subsequent to the insertion thereof into the aperture when the dynamic fluid is in the second state such that lateral movement of the surgical object with respect to the longitudinal axis is substantially limited.

A method of performing a minimally invasive surgical procedure, comprising the steps of:
inserting a surgical access member into a percutaneous access point within tissue to gain access to an underlying body cavity;
introducing a surgical object within the surgical access member and through a valve disposed within the access member, the valve including at least one internal cavity configured to retain dynamic fluid;
stimulating the dynamic fluid to cause the dynamic fluid to transition from a first state having a first viscosity to a second state having a second viscosity greater than the first viscosity to assist in stabilizing the surgical object relative to the longitudinal axis; and
performing a procedure with the surgical object.

### BRIEF DESCRIPTION OF THE DRAWINGS

Various embodiments of the present disclosure are described herein below with references to the drawings, wherein:

**FIG. 1** is a side schematic view of a valve in accordance with the principles of the present disclosure disposed within a cannula assembly;

**FIG. 2A** is a perspective of the valve of **FIG. 1** shown in a first condition;

**FIG. 2B** is a side cross-sectional view of the valve of **FIGS. 1-2** shown in the first condition;

**FIG. 3** is a perspective view of one embodiment of the valve of **FIG. 1** having a substantially conical configuration;

**FIG. 4** is a side cross-sectional view of the valve of **FIGS. 1-2** shown in a second condition with a surgical instrument inserted therethrough in substantial alignment with a longitudinal axis defined by an aperture formed in the valve; and

**FIG. 5** is a side cross-sectional view of the valve of **FIGS. 1-2** shown in a second condition with the surgical instrument inserted therethrough in misalignment with the longitudinal axis.

### DETAILED DESCRIPTION OF THE EMBODIMENTS

In the drawings and in the description which follows, in which like references numerals identify similar or identical elements, the term "proximal" will refer to the end of the apparatus which is closest to the clinician, while the term "distal" will refer to the end which is furthest from the clinician, as is traditional and known in the art. Additionally, use of the term "surgical object" herein below should be understood to include any surgical object or instrument that may be employed during the course of surgical procedure, including but not being limited to an obturator, a surgical stapling device, or the like.

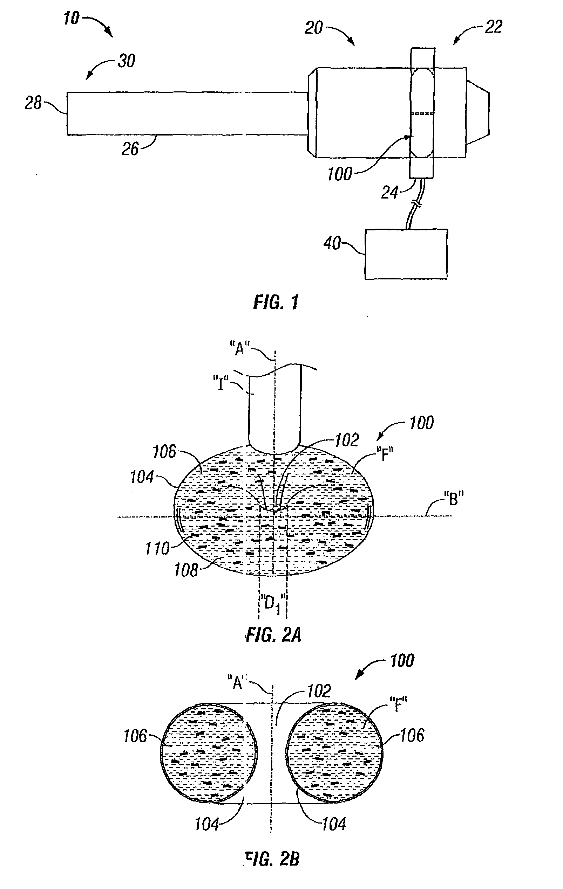

With reference to **FIG. 1****,** a surgical access assembly **10** is illustrated. Access assembly **10** includes a reusable surgical access apparatus, e.g., cannula assembly **20,** and a stimulation member **40.**

At a proximal end **22,** cannula assembly **20** includes a housing **24.** Housing **24** is configured to accommodate a valve **100,** and may be any structure suitable for this intended purpose. Further information regarding valve housing **24** may be obtained through reference to commonly owned U.S Patent No. 7,169,130 to Exline et al.***,*** the entire contents of which are hereby incorporated by reference.

Extending distally from housing **24** is a shaft **26** that is configured for the internal receipt of a surgical object (not shown). Shaft 26 defines an opening **28** at its distal end **30** that is dimensioned to allow the surgical object (not shown) to pass therethrough, thereby facilitating percutaneous access to a patient's internal cavities with the surgical object.

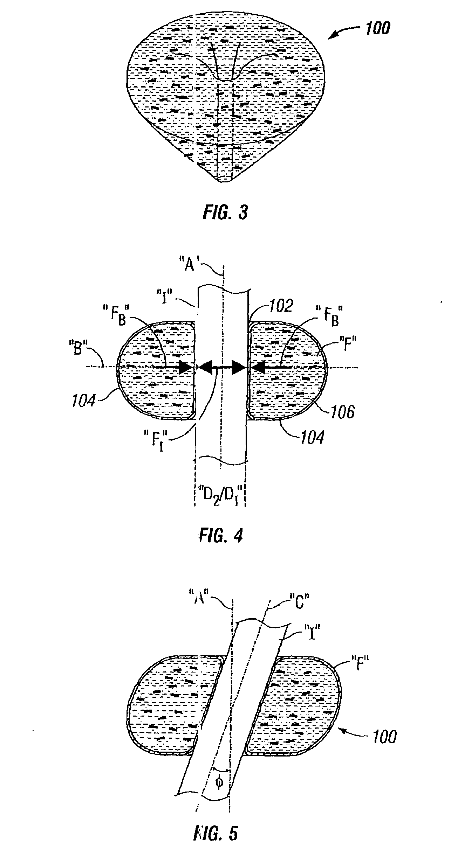

Referring now to **FIGS. 2A-4****,** the valve **100** that is the subject of the present disclosure will be discussed. Valve **100** includes an aperture **102** that extends therethrough along a longitudinal axis "A". Aperture **102** is dimensioned to removably receive the surgical object **"I"** such that a substantially fluid-tight seal is formed therewith. In one embodiment, valve **100** may define a substantially torroidal configuration (FIGS. **2A-2B****),** whereas in an alternate embodiment, valve **100** may define a substantially conical configuration **(****FIG. 3****)** that extends distally to thereby facilitate the insertion of surgical object "I" within valve **100.**

Valve **100** includes an outer membrane **104** that defines an internal cavity **106.** Outer membrane **104** may be formed of any suitable biocompatible material that is at least semi-resilient in nature. Forming outer membrane **104** of such a material allows outer membrane **104,** and consequently aperture 102, to deform or stretch upon the introduction of surgical object "I" thereto, as discussed in further detail below.

Cavity **106** is configured to retain a fluid **"F"** therein, which provides a measure of structure and rigidity to valve **100.** However, fluid **"F"** may be displaced within cavity **106** such that valve **100,** and aperture **102,** are allowed to reversibly and resiliently deform so as to accommodate surgical object "I" upon the insertion thereof. The resiliency of valve **100** permits varying degrees of deformation during use, thereby allowing for the use of valve **100** in connection with surgical objects that may vary in size and facilitating the maintenance of a substantially fluid-tight seal therewith.

Prior to the insertion of surgical object "I", valve **100** is at rest and in a first (or initial) condition thereof. In the first condition, aperture **102** defines a first dimension **"D₁"** measured along an axis **"B"** that is transverse in relation to the longitudinal axis "A" along which aperture **102** extends. In one embodiment, dimension **"D₁"** may be approximately equal to zero such that aperture **102** is substantially closed when valve **100** is in the fist condition, thereby preventing the escape of any insufflation gas through valve **100** in the absence of surgical object **"I".**

As seen in **FIG. 4****,** upon its insertion, surgical object **"I"** exerts a force **"F_{I}"** upon outer membrane **104** at aperture **102** that is directed outwardly with respect to longitudinal axis **"A",** i.e., along transverse axis "B". Force **"F_{I}"** causes outer membrane **104,** and aperture **102,** to outwardly deform or stretch, thereby displacing fluid "F" within cavity **106** and transitioning valve **100** into a second (or deformed) condition. The natural tendency of the resilient material comprising outer membrane **104,** as well as that of the displaced fluid "F", to return valve **100** to first condition creates a biasing force **"F_{B}"** that is directed inwardly with respect to longitudinal axis "A". The magnitude of biasing force **"F_{B}"** is directly related to that of force **"F_{I}"** and acts upon surgical object "I". In the second condition, aperture **102** defines a second, larger transverse dimension **"D₂"** relative to **"D₁"** that substantially approximates the outer dimension **"D_{I}"** of surgical object "I", thereby creating a substantially fluid-tight seal between valve **100** and surgical object "I" at aperture **102** such that the escape of insufflation gas about surgical object "I" through valve **100** is substantially prevented. The diameter **"D_{I}"** of surgical object "I", and thus the transverse dimension **"D₂"** of aperture **102** in the second condition, will generally lie within the range of approximately 5mm to approximately 15mm, as is conventional to the art, although the use of substantially larger and smaller surgical objects in connection with valve 100 is also within the scope of the present disclosure.

The fluid **"F"** retained within cavity **106** is a dynamic fluid in that it is adapted to transition from a first state to a second state upon the introduction of a stimulus thereto. In its first state, fluid **"F"** defines a first set of physical attributes, including but not being limited to viscosity and density, that allow the displacement of fluid **"F"** within cavity **106**, whereas in the second state, fluid **"F"** defines a second, dissimilar set of physical attributes, e.g., a greater viscosity and a greater density, that substantially limit the displacement of fluid **"F".** Accordingly, as fluid **"F"** transitions from its first state to its second state, fluid **"F"** and, consequently valve **100,** are considerably rigidified.

In one embodiment of valve **100,** fluid **"F"** may be a "smart fluid" comprised of one or more carrier fluids **108** having a plurality of particles **110** dispersed therein. Suitable carrier fluids **108** may include, but are not limited to, organic liquids, especially non-polar organic liquids, such as silicone oils, mineral oils, paraffin oils, silicone copolymers, or any combination thereof. Particles **110** may be responsive to an electric field, such that fluid **"F"** may be characterized as an electrorheological fluid, or a magnetic field, such that fluid **"F"** may be characterized as a magnetorheological fluid, a paramagnetic fluid, or a ferrofluid. Particles **110** may include one or more of iron, iron alloys such as those including aluminum, silicon, cobalt, nickel, vanadium, molybdenum, chromium, tungsten, manganese and/or copper, iron oxides, including Fe₂O₃ and Fe₃O₄, iron nitride, iron carbide, carbonyl iron, nickel and alloys thereof, cobalt and alloys thereof, chromium dioxide, stainless steel, silicon steel, or carbonyl iron.

As indicated above, surgical access assembly **10** includes a stimulation member **40.** Stimulation member **40** is operatively associated with valve **100,** and when activated, generates the stimulus which transitions fluid **"F"** from the first state to the second state. Stimulation member **40,** therefore, allows the clinician to selectively regulate the state of fluid **"F".** As previously discussed, fluid **"F"** may include particles that are responsive to, e.g., electrical or magnetic energy fields, and accordingly, stimulation member **40** may be any member suitable for the intended purpose of generating such energy fields.

Referring now to **FIGS. 1-4**, the use and function of valve **100** will be discussed in conjunction with a surgical portal apparatus, e.g., cannula assembly **20.** Initially, the target work site is insufflated with a suitable biocompatible gas, e.g., CO₂ gas, such that a larger internal work space may be created within the patient, thereby providing greater access to internal organs, cavities, tissues, etc. The insufflation may be performed with an insufflation needle or similar device, as is conventional in the art. Following insufflation, cannula assembly **20** is placed within a percutaneous access point in the patient's tissue (not shown), either preexisting or created by the clinician using an obturator (not shown) or the like, as is known in the art. Subsequently, surgical object "I" is inserted into cannula assembly **20.**

Upon the introduction of surgical object **"I"** to valve **100,** valve **100** transitions from the first condition to the second condition, forming a substantially fluid-tight seal with surgical object **"I".** It should be noted that, prior to the introduction of surgical object **"I"** to valve **100,** fluid **"F"** is in its first state. Accordingly, upon the insertion of surgical object **"I",** and during the lateral manipulation thereof within valve **100** along transverse axis **"B",** fluid **"F"** may be displaced within cavity **106.** However, should it become desirably to maintain a particular orientation of surgical object **"I",** e.g., vertically, such that surgical object **"I"** is aligned with longitudinal axis "A" **(****FIG. 4****),** or along an axis **"C"** that forms an angle 0 therewith **(****FIG. 5****),** the clinician may actuate stimulation member **40,** thereby transitioning fluid "F" from the first state to the second state. As discussed above, in the second state, fluid **"F"** is substantially rigid, defining physical attributes that substantially limit the displacement thereof. Consequently, any further displacement of surgical object **"I"** within valve **100** is also substantially limited, thereby preserving the orientation of surgical object **"I".** When it is no longer necessary or desirable to limit the movement of surgical object **"I",** stimulation member **40** may be deactivated such that fluid **"F"** may return to its first state, thereby once again permitting the displacement of fluid **"F",** and likewise, the lateral movement of surgical object **"I"** within valve **100.** The remainder of the surgical procedure may then be carried out, after which surgical object **"I"** may be removed from surgical access assembly **10,** surgical access assembly **10** may be removed from the access point in the patient's tissue **"T",** and the access point may be closed.

Although the illustrative embodiments of the present disclosure have been described herein with reference to the accompanying drawings, the above description, disclosure, and figures should not be construed as limiting, but merely as exemplifications of particular embodiments. It is to be understood, therefore, that the disclosure is not limited to those precise embodiments, and that various other changes and modifications may be effected therein by one skilled in the art without departing from the scope or spirit of the disclosure.

## Claims

1. A surgical access instrument for use during the course of a minimally invasive surgical procedure, which comprises:
an access member defining a longitudinal axis and having a longitudinal opening for reception and passage of a surgical object;
a valve membrane mounted to the access member, the valve membrane comprising a biocompatible material and defining at least one internal cavity, the valve membrane permitting passage of the surgical object; and
a dynamic fluid disposed within the internal cavity, the dynamic fluid being adapted to transition from a first state to a second state upon exposure thereof to a stimulus to assist in stabilizing the surgical object relative to the access member.

2. The surgical access instrument of claim 1, including a stimulation member in communication with the dynamic fluid, the stimulation member being adapted to selectively generate the stimulus.

3. The surgical access instrument of claim 2, wherein the stimulation member is adapted to selectively generate an electrical field.

4. The surgical access instrument of claim 2, wherein the stimulation member is adapted to selectively generate a magnetic field.

5. The surgical access instrument of claim 1, wherein the dynamic fluid is selected from the group consisting of an electrorheological fluid, a magnetorheological fluid, and a ferrofluid.

6. The surgical access instrument of claim 1, wherein the dynamic fluid defines a first viscosity in the first state and a second viscosity in the second state, the second viscosity being greater than the first viscosity.

7. The surgical access instrument of claim 1, wherein the valve membrane includes an aperture extending therethrough, the valve membrane comprising a semi-resilient material such that the surgical valve may resiliently transition between first and second conditions upon the insertion of at least one surgical object into the aperture, the aperture defining a first transverse dimension in the first condition and a second transverse dimension in the second condition, the second transverse dimension being greater than the first transverse dimension.

8. The surgical access instrument of claim 7, wherein the valve membrane defines a substantially torroidal configuration.

9. The surgical access instrument of claim 7, wherein the valve membrane is adapted to apply a force to the surgical object subsequent to the insertion thereof into the aperture when the dynamic fluid is in the second state such that lateral movement of the surgical object with respect to the longitudinal axis is substantially limited.

10. A surgical access member for use in a method of performing a minimally invasive surgical procedure wherein the method comprises the steps of:
inserting the surgical access member into a percutaneous access point within tissue to gain access to an underlying body cavity;
introducing a surgical object within the surgical access member and through a valve disposed within the access member, the valve including at least one internal cavity configured to retain dynamic fluid;
stimulating the dynamic fluid to cause the dynamic fluid to transition from a first state having a first viscosity to a second state having a second viscosity greater than the first viscosity to assist in stabilizing the surgical object relative to the longitudinal axis; and
performing a procedure with the surgical object.
